# EUROPÄISCHE PATENTANMELDUNG

(11) **EP 3 409 323 A1**
(43) Veröffentlichungstag der Anmeldung: **05.12.2018**
(21) Anmeldenummer: 18175455.7
(22) Anmeldetag: 01.06.2018
(51) Int. Cl.: A61Q 9/04, A61K 8/73, A61K 8/97, A61K 8/19, A61K 8/23, A61K 8/02, A61K 8/9783

(54) **ZUSAMMENSETZUNG ZUR HAARENTFERNUNG**

(30) Priorität: 01.06.2017 DE 202017103314 U
(71) Anmelder: Azimian, Behzad, 87700 Memmingen (DE); Azimian, Vincent, 87700 Memmingen (DE)
(72) Erfinder: Azimian, Behzad, 87700 Memmingen (DE)
(74) Vertreter: Rings, Rolf

(57) **Zusammenfassung**

Zusammensetzung zur direkten oder indirekten körperlichen Anwendung auf der Haut oder dem Haar, insbesondere zum Entfernen von unerwünschtem Haarwuchs, mit mindestens einem Wirkstoffhauptbestandteil und mindestens einem zusätzlichem Wirkstoff, wobei der Wirkstoffhauptbestandteil aus einem oder mehreren von den Stoffen ausgewählt aus Calciumcarbonat, Calciumhydroxid, Calciumthioglykalat, Strontiumhydroxid, Sulfur, Strontium, Schwefel, Weizenstärke oder Bentonit in vordefinierten Anteilen und Mengen besteht, wobei sowohl der Wirkstoffhauptbestandteil als auch mindestens einer der zusätzlichen Wirkstoffe in Pulverform vorliegen, und dass mindestens einer der zusätzlichen Wirkstoffe ein pulverförmiges Aloe-Vera-Extrakt ist oder enthält.

## Beschreibung

Die vorliegende Erfindung betrifft eine Zusammensetzung zur direkten oder indirekten körperlichen Anwendung auf der Haut oder dem Haar zum Entfernen von unerwünschtem Haarwuchs. Derartige Enthaarungsmittel werden aus kosmetischen oder medizinischen Gründen eingesetzt, um unerwünschte Haare auf einem Körper lokal oder großflächig zu entfernen.

Zum Entfernen solch unerwünschtem Haarwuchs wurden bisher verschiedene Methoden und Körperpflegeprodukte eingesetzt, wie zum Beispiel ein Rasieren mit Rasierklingen, Enthaarungscremes oder Enthaarungslotionen, Wachsentfernungen, laserbasierte Enthaarungsvorrichtungen oder dergleichen. Bei diesen bisher angewendeten Methoden zum Entfernen vom Haarwuchs besteht ein Nachteil dahingehend, dass beispielsweise beim Rasieren kleinere oder größere Schnittwunden oder Hautreizungen hervorgerufen werden können. Auch bei einer Enthaarung mittels Wachs oder mittels Laser können erhebliche Hautreizungen oder Ausschläge auf der Haut verursacht werden. Bei den technischen Haarentfernungsmethoden mittels Elektrolyse oder Laser ist die Anwendung der Enthaarung äußerst kostenintensiv. Außerdem können diese Methoden zu Narben und langfristigen Schädigungen des Körpers eines Nutzers führen.

Vor diesem Hintergrund ist es die Aufgabe der vorliegenden Erfindung, ein neuartiges Enthaarungsmittel in Form einer Zusammensetzung zur körperlichen Anwendung auf der Haut und dem Haar bereitzustellen, welche ein geringes Schädigungs- und Reizungspotential aufweist und welche mit überschaubaren Kosten eine effektive Enthaarung auf möglichst andere Art und Weise erlaubt.

Diese Aufgabe wird mit einer Zusammensetzung mit den Merkmalen des Anspruchs 1 gelöst. Vorteilhafte Ausgestaltung und Weiterbildung der erfindungsgemäßen Zusammensetzung als Enthaarungsmittel sind in den abhängigen Ansprüchen definiert.

Die erfindungsgemäße Zusammensetzung dient zur Haarentfernung und weist mindestens einen Wirkstoffhauptbestandteil und mindestens einen zusätzlichen Wirkstoff auf, wobei der Wirkstoffhauptbestandteil aus einem oder mehreren von den Stoffen ausgewählt aus Calciumcarbonat, Calciumhydroxid, Calciumthioglykalat, Strontiumhydroxid, Sulfur, Strontium, Schwefel, Weizenstärke oder Bentonit oder einer Mischung davon in vordefinierten Anteilen und Mengen besteht, wobei die Erfindung dadurch gekennzeichnet ist, dass sowohl der Wirkstoffhauptbestandteil als auch mindestens einer der zusätzlichen Wirkstoffe in Pulverform vorliegen und wobei mindestens einer der zusätzlichen Wirkstoffe ein pulverförmiges Aloe-Vera-Extrakt ist oder enthält.

Mit solch einer Zusammensetzung liegt ein pulverförmiges Enthaarungsmittel vor, dass auf Basis von hauptsächlich natürlichen Bestandteilen zusammengesetzt wird. Das Enthaarungsmittel, welches aus dieser erfindungsgemäßen Zusammensetzung besteht, ist damit nicht nur in der Benutzerfreundlichkeit und Ergebniseffizienz verbessert. Zudem lassen sich mit solch einer Zusammensetzung die unerwünschten Reizungen, Beschädigungen oder Hautirritationen effektiv vermeiden. Die Zusammensetzung enthält keinerlei ungesunde Chemikalien oder künstliche Stoffe. Auch ist ein mechanischer Eingriff an der Hautoberfläche, wie zum Beispiel beim Rasieren, nicht erforderlich. Erfindungsgemäß wird als mindestens ein zusätzlicher Wirkstoff ein pulverförmiges Aloe-Vera-Extrakt vorgesehen, welches in dem zusätzlichen Wirkstoff enthalten ist oder daraus besteht. Die Zusammensetzung insgesamt liegt somit in Form eines Pulvers vor. Für die Anwendung wird die pulverförmige Zusammensetzung mit dem Aloe-Vera-Bestandteil mit Wasser angerührt, auf die Haut aufgetragen und nach einer gewissen Wartezeit wird die Masse dann mit reichlich Wasser abgespült. Die Wirkstoffe des Wirkstoffhauptbestandteils und des zusätzlichen Wirkstoffs in Form von Aloe-Vera in Pulverform haben in der Zwischenzeit die Haare weitestgehend losgelöst und damit entfernt, sodass sie zusammen mit der Masse mit Wasser abgespült werden können.

Die erfindungsgemäße Zusammensetzung ist sehr effizient und in gesundheitlicher Hinsicht weit benutzerfreundlicher als es bei bisherigen solch oben beschriebenen Methoden der Fall war. Unerwünschte Reizungen, Rötungen oder Schwellungen der Haut eines Nutzers sind im Wesentlichen vermieden. Der zusätzliche Wirkstoff in Form von pulverförmigem Aloe-Vera hat außerdem den Vorteil, dass die Zusammensetzung auch Juckreiz mindernd ist, entzündungshemmend und feuchtigkeitsbewahrend ist. Überraschenderweise hat sich bei Untersuchungen gezeigt, dass besonders als Pulver das Aloe-Vera-Extrakt überraschende Vorteile bei einer Enthaarung bietet, welche so in den bisher bekannten Methoden und Arten nicht gegeben waren.

Gemäß einer vorteilhaften Ausgestaltung der Erfindung ist als zusätzlicher Wirkstoff in der Zusammensetzung Aloe Vera in einem Anteil von 35 Gew.-%, vorzugsweise von mindestens 50 bis 75 Gew.-% enthalten. In diesen mindesten Mengen und Anteilen von Aloe Vera in der Zusammensetzung wurden besonders gute Ergebnisse hinsichtlich der Verträglichkeit und der Effizienz einer Haarentfernung erreicht.

Gemäß einer weiteren vorteilhaften Ausgestaltung der Erfindung ist als ein weiter zusätzlicher Wirkstoff ein natürliches Aroma oder mehrere natürliche Aromen vorgesehen. Insbesondere sind natürliche Aromen in Form von Kräutern, Früchten, Blüten, Wurzeln oder Blättern vorgesehen. Die Zusammensetzung ist somit durch natürliche Aromen angereichert, welche zusätzliche Vorteile hinsichtlich der Verträglichkeit und des Nutzungskomforts bieten: insbesondere können natürliche Aromen beispielsweise in Form von Mischungen von mehreren Kräutern in der Zusammensetzung enthalten sein. Die natürlichen Kräuter sind vorzugsweise derart ausgewählt, dass sie die Haarentfernung unterstützen und die Reizung und Beeinträchtigung der Haut beim Haarentfernen weiter minimieren. Auch lassen sich so auf überraschend einfache Weise Haarentfernungsmittel realisieren, bei denen die Geruchsentwicklung weitgehend unterdrückt ist, beispielsweise indem Blüten oder Früchte in Form von Pulvern, Extrakten oder in flüssigen Form oder körniger Form hinzugefügt sind.

Nach einer alternativen Ausgestaltung der Erfindung ist/sind als zusätzlicher Wirkstoff ein oder mehrere naturidentische Aromen vorgesehen. Mit diesen naturidentischen Aromen kann die Wirkung und Anwendung der Zusammensetzung weiter optimiert werden.

Gemäß einer besonders bevorzugten Ausgestaltung der Erfindung ist als ein zusätzlicher Wirkstoff ein pflanzlicher Wirkstoff auf der Basis der Damaszener Rose (lat.: Rose damascena) oder von Henna aus dem Hennastrauch vorgesehen.

Gemäß einer weiteren vorteilhaften Ausgestaltung der Erfindung kann zusätzlich zu natürlichen Aromen auch ein weiterer zusätzlicher Wirkstoff in Form von mindestens einem synthetischen Duft-/Aromastoff beigemengt sein. Auf diese Weise lassen sich die unangenehmen Geruchsentwicklungen auf Basis der Wirkstoffhauptbestandteile weiter effektiv reduzieren oder ganz vermeiden.

Gemäß einer weiteren vorteilhaften Ausgestaltung der Erfindung ist die pulverförmige Aloe Vera in der Zusammensetzung ein sprüh- oder gefriergetrocknetes Aloe-Vera-Extrakt.

Gemäß einer weiteren vorteilhaften Ausgestaltung der Erfindung umfasst die Zusammensetzung des Weiteren ein pulverförmiges Bindemittel. Mit solch einem pulverförmigen Bindemittel wird die Zusammensetzung besser auf die Haut bei der Anwendung auftragbar. Auch lassen sich mit solchen zusätzlichen Bindemittel die einzelnen Bestandteile der Zusammensetzung besser miteinander mischen und in einheitlicher Form bereitstellen.

Gemäß einer weiteren vorteilhaften Ausgestaltung der Erfindung ist die Zusammensetzung im Wesentlichen frei von Konservierungsstoffen, synthetischen keimhemmenden Substanzen, Emulgatoren und/oder Stabilisatoren. Bei bisher bekannten derartigen Enthaarungsmittel auf Basis von beispielsweise gelförmigen Aloe-Vera-Mitteln oder ähnlichen bestand ein erheblicher Nachteil darin, dass die Haltbarkeit stark beschränkt ist und die beispielsweise auch gekühlt nur wenige Monate aufbewahrt werden konnten. Außerdem waren derartige bisher bekannte Zusammensetzung zur Haarentfernung in der Regel mit zusätzlichen synthetischen keimhemmenden Substanzen versehen, welche die beschriebenen Nachteile hinsichtlich einer physischen oder chemischen Reizung der Haut hervorrufen können. Mit der Erfindung ist so weitgehend ein natürliches Mittel zur Haarentfernung in Form der beschriebenen Zusammensetzung mit Aloe Vera in Pulverform bereitgestellt, welches in vielerlei Hinsicht Vorteile hat: nicht nur die chemische Unbedenklichkeit und medizinische Verträglichkeit auch die Effizienz in der Haarentfernung wird mit den Mitteln deutlich erhöht, da das pulverförmige Aloe-Vera-Extrakt sich als sehr wirksamer zusätzlicher Wirkstoff in solch einer Zusammensetzung überraschend erwiesen hat wie sie in den Ansprüchen angegeben ist.

Gemäß einer weiteren vorteilhaften Ausgestaltung der Erfindung ist in der Zusammensetzung ein pulverförmiges Aloe-Vera-Extrakt aus der Pflanzenart Aloe barbadensis Miller vorhanden.

Die Erfindung ist nicht auf die beschriebene Zusammensetzung, Anteile und Mengen beschränkt. Sie umfasst sämtliche Varianten und Abwandlung der Zusammensetzung im Rahmen der nachfolgenden Ansprüche, sofern als mindestens ein zusätzlicher Wirkstoff ein pulverförmiges Aloe-Vera-Extrakt vorliegt.

## Patentansprüche

1. Zusammensetzung zur direkten oder indirekten körperlichen Anwendung auf der Haut oder dem Haar, insbesondere zum Entfernen von unerwünschtem Haarwuchs, mit mindestens einem Wirkstoffhauptbestandteil und mindestens einem zusätzlichem Wirkstoff, wobei der Wirkstoffhauptbestandteil aus einem oder mehreren von den Stoffen ausgewählt aus Calciumcarbonat, Calciumhydroxid, Calciumthioglykalat, Strontiumhydroxid, Sulfur, Strontium, Schwefel, Weizenstärke oder Bentonit in vordefinierten Anteilen und Mengen besteht, **dadurch gekennzeichnet, dass** sowohl der Wirkstoffhauptbestandteil als auch mindestens einer der zusätzlichen Wirkstoffe in Pulverform vorliegen, und dass mindestens einer der zusätzlichen Wirkstoffe ein pulverförmiges Aloe-Vera-Extrakt ist oder enthält.

2. Zusammensetzung nach Anspruch 1, **dadurch gekennzeichnet, dass** als zusätzlicher Wirkstoff pulverförmiges Aloe Vera in einem Anteil von mindestens 35 Gew.-%, vorzugsweise von mindestens 50 bis 75 Gew.-% enthalten ist.

3. Zusammensetzung nach Anspruch 1 oder 2, **dadurch gekennzeichnet, dass** als weiterer zusätzlicher Wirkstoff natürliche Aromen, insbesondere Aromen in Form von Kräutern, Früchten, Blüten, Wurzeln oder Blättern, vorgesehen sind.

4. Zusammensetzung nach Anspruch 3, **dadurch gekennzeichnet, dass** die natürlichen Aromen der Zusammensetzung in flüssiger, trockener oder körniger Form, insbesondere in Form von ätherischen Ölen, Pasten, Harzen oder Duftstoffextrakten, zugefügt sind.

5. Zusammensetzung nach einem der vorangegangenen Ansprüche, **dadurch gekennzeichnet, dass** als ein weiterer zusätzlicher Wirkstoff mindestens ein synthetischer Duft-/Aroma-Stoff vorhanden ist.

6. Zusammensetzung nach einem der vorangegangenen Ansprüche, **dadurch gekennzeichnet, dass** das pulverförmige Aloe Vera ein sprüh- oder gefriergetrocknetes Aloe-Vera-Extrakt ist.

7. Zusammensetzung nach einem der vorangegangenen Ansprüche, **dadurch gekennzeichnet, dass** die Zusammensetzung ein pulverförmiges Bindemittel umfasst.

8. Zusammensetzung nach einem der vorangegangenen Ansprüche, **dadurch gekennzeichnet, dass** die Zusammensetzung im Wesentlichen frei von Konservierungsstoffen, synthetischen keimhemmenden Substanzen, Emulgatoren und/oder Stabilisatoren ist.

9. Zusammensetzung nach einem der vorangegangenen Ansprüche, **dadurch gekennzeichnet, dass** das pulverförmige Aloe Vera ein Extrakt aus der Pflanzenart Aloe barbadensis Miller ist.
